(19) Europäisches Patentamt
European Patent Office
Office européen des brevets

(11) Veröffentlichungsnummer: **0 173 312**
**A1**

(12) **EUROPÄISCHE PATENTANMELDUNG**

(21) Anmeldenummer: **85110823.3**

(22) Anmeldetag: **19.08.85**

(51) Int. Cl.⁴: **C 07 D 239/42**, C 07 D 251/42, C 07 D 251/48, C 07 D 253/04, C 07 D 253/06, C 07 D 257/08, C 07 D 213/75, C 07 C 143/828, C 07 C 161/04, A 01 N 47/36

(30) Priorität: **30.08.84 DE 3431917**

(43) Veröffentlichungstag der Anmeldung: **05.03.86 Patentblatt 86/10**

(84) Benannte Vertragsstaaten: **AT BE CH DE FR GB IT LI NL SE**

(71) Anmelder: **BAYER AG, Konzernverwaltung RP Patentabteilung, D-5090 Leverkusen 1 Bayerwerk (DE)**

(72) Erfinder: **Diehr, Hans-Joachim, Dr., Höhe 35, D-5600 Wuppertal 1 (DE)**
Erfinder: **Fest, Christa, Dr., Im Johannistal 20, D-5600 Wuppertal 1 (DE)**

(72) Erfinder: **Kirsten, Rolf, Dr., Carl-Langhans-Strasse 27, D-4019 Monheim (DE)**
Erfinder: **Kluth, Joachim, Dr., Kurt-Schumacher-Strasse 9, D-4018 Langenfeld (DE)**
Erfinder: **Müller, Klaus-Helmut, Dr., Bockhackstrasse 55, D-4000 Düsseldorf 13 (DE)**
Erfinder: **Pfister, Theodor, Dr., Lichtenberger Strasse 30, D-4019 Monheim (DE)**
Erfinder: **Priesnitz, Uwe, Dr., Severinstrasse 58, D-5650 Solingen 1 (DE)**
Erfinder: **Riebel, Hans-Jochem, Dr., In der Beek 92, D-5600 Wuppertal 1 (DE)**
Erfinder: **Roy, Wolfgang, Dr., Walter-Kolb-Strasse 47, D-4018 Langenfeld (DE)**
Erfinder: **Santel, Hans-Joachim, Dr., Gerstenkamp 19, D-5000 Köln 80 (DE)**
Erfinder: **Schmidt, Robert R., Dr., Im Waldwinkel 110, D-5060 Bergisch-Gladbach 2 (DE)**
Erfinder: **Eue, Ludwig, Dr., Paul-Klee-Strassse 36, D-5090 Leverkusen (DE)**
Erfinder: **Kysela, Ernst, Dr., Virchowstrasse 14, D-5060 Bergisch-Gladbach 1 (DE)**

(54) **1-(2-Trifluormethoxy-phenylsulfonyl)-3-heteroaryl-(thio)-harnstoffe.**

(57) Die Erfindung betrifft neue 1-(2-Trifluormethoxy-phenylsulfonyl)- 3-heteroaryl-(thio)harnstoffe der allgemeinen Formel (I)

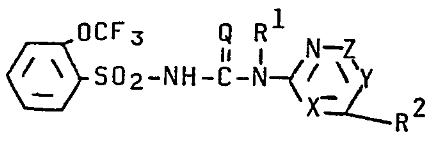

(I)

in welcher
Q für Sauerstoff oder Schwefel steht,
$R^1$ für Wasserstoff, $C_1$–$C_4$-Alkyl oder Benzyl steht,
$R^2$ für Wasserstoff, Halogen, Hydroxy $C_1$–$C_4$-Alkyl, $C_1$–$C_4$-Halogenalkyl, $C_1$–$C_4$-Alkoxy, $C_1$–$C_4$-Halogenalkoxy, $C_1$–$C_4$-Alkylthio, $C_1$–$C_4$-Halogenalkylthio, Amino, $C_1$–$C_4$-Alkylamino oder Di-($C_1$–$C_4$-alkyl)-amino steht,
X für Stickstoff oder eine -CH-Gruppierung steht,
Y für Stickstoff oder eine -$CR^3$-Gruppierung steht, worin
$R^3$ für Wasserstoff, Halogen, $C_1$–$C_4$-Alkyl, ($C_1$–$C_2$-Alkyl)-carbonyl oder ($C_1$–$C_2$-Alkoxy)-carbonyl steht,
Z für Stickstoff oder eine -$CR^4$-Gruppierung steht, worin
$R^4$ für Wasserstoff, Halogen, Hydroxy, $C_1$–$C_4$-Alkyl, $C_1$–$C_4$-Alkoxy, $C_1$–$C_4$-Alkylthio, $C_1$–$C_4$-Alkylamino oder Di-($C_1$–$C_4$-alkyl)-amino steht, Verfahren zu ihrer Herstellung und ihre Verwendung als Herbizide.

EP 0 173 312 A1

0 173 312

BAYER AKTIENGESELLSCHAFT
Konzernverwaltung RP
Patentabteilung

5090 Leverkusen, Bayerwerk
Bi/mö
(Ib)

1-(2-Trifluormethoxy-phenylsulfonyl)-3-heteroaryl-(thio)-harnstoffe

Die Erfindung betrifft neue 1-(2-Trifluormethoxy-phenylsulfonyl)-3-heteroaryl-(thio)harnstoffe, mehrere Verfahren zu ihrer Herstellung und ihre Verwendung als Herbizide.

Es ist bekannt, daß bestimmte 1-Arylsulfonyl-3-heteroarylharnstoffe, wie z. B. 1-(2-Methoxy-phenylsulfonyl)-3-(4,6-dimethyl-pyrimidin-2-yl)-harnstoff, herbizid wirksam sind. Die Wirkung dieser Verbindungen ist jedoch nicht immer ganz befriedigend (vergl. US-PS 4 169 719).

Es wurden nun neue 1-(2-Trifluormethoxy-phenylsulfonyl)-3-heteroaryl-(thio)harnstoffe der allgemeinen Formel (I)

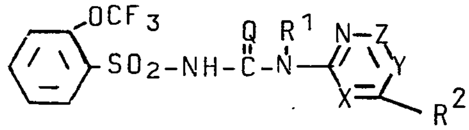

(I)

in welcher

Q für Sauerstoff oder Schwefel steht,

$R^1$ für Wasserstoff, $C_1$-$C_4$-Alkyl oder Benzyl steht,

$R^2$ für Wasserstoff, Halogen, Hydroxy, $C_1$-$C_4$-Alkyl, $C_1$-$C_4$-Halogenalkyl, $C_1$-$C_4$-Alkoxy, $C_1$-$C_4$-Halogenalkoxy, $C_1$-$C_4$-Alkylthio, $C_1$-$C_4$-Halogenalkylthio, Amino, $C_1$-$C_4$-Alkylamino oder Di-($C_1$-$C_4$-alkyl)-amino steht,

X für Stickstoff oder eine -CH-Gruppierung steht,

Y für Stickstoff oder eine $-CR^3$-Gruppierung steht,

worin

$R^3$ für Wasserstoff, Halogen, $C_1$-$C_4$-Alkyl, ($C_1$-$C_2$-Alkyl)-carbonyl oder ($C_1$-$C_2$-Alkoxy)-carbonyl steht,

Z für Stickstoff oder eine $-CR^4$-Gruppierung steht,

worin

$R^4$ für Wasserstoff, Halogen, Hydroxy, $C_1$-$C_4$-Alkyl, $C_1$-$C_4$-Alkoxy, $C_1$-$C_4$-Alkylthio, $C_1$-$C_4$-Alkylamino oder Di-($C_1$-$C_4$-alkyl)-amino steht,

gefunden.

Man erhält die neuen 1-(2-Trifluormethoxy-phenylsulfonyl)--3-heteroaryl-(thio)harnstoffe der Formel (I), wenn man

(a) 2-Trifluormethoxy-phenylsulfonyl-iso(thio)cyanate der

der Formel (II)

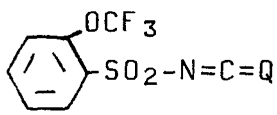 (II)

in welcher

Q für Sauerstoff oder Schwefel steht,

mit Heteroarylaminen der Formel (III)

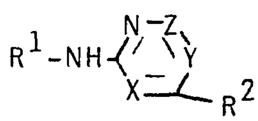 (III)

in welcher

$R^1$, $R^2$, X, Y und Z die oben angegebenen Bedeutungen haben,

gegebenenfalls in Gegenwart von Verdünnungsmitteln und gegebenenfalls in Gegenwart von Katalysatoren umsetzt,

oder

(b) 2-Trifluormethoxy-benzolsulfonsäureamid der Formel (IV)

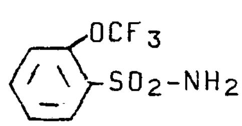 (IV)

mit N-Heteroaryl-urethanen der Formel (V)

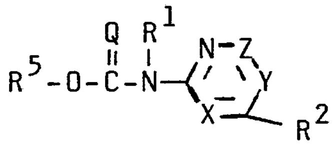

(V)

in welcher

$R^1$, $R^2$, Q, X, Y und Z die oben angegebenen Bedeutungen haben und

$R^5$ für $C_1$-$C_4$-Alkyl, Benzyl oder Phenyl steht,

gegebenenfalls in Gegenwart von Verdünnungsmitteln und gegebenenfalls in Gegenwart von Säureakzeptoren umsetzt,

oder

(c) N-(2-Trifluormethoxy-phenylsulfonyl)-urethane der Formel (VI)

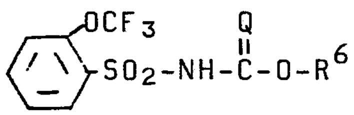

(VI)

in welcher

Q für Sauerstoff oder Schwefel steht und

$R^6$ für $C_1$-$C_4$-Alkyl, Benzyl oder Phenyl steht,

mit Heteroarylaminen der Formel (III)

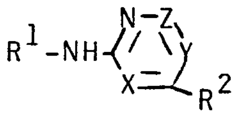

(III)

in welcher

$R^1$, $R^2$, X, Y und Z die oben angegebenen Bedeutungen haben,

gegebenenfalls in Gegenwart von Verdünnungsmitteln und gegebenenfalls in Gegenwart von Säureakzeptoren umsetzt.

Die neuen 1-(2-Trifluormethoxy-phenylsulfonyl)-3-heteroaryl-(thio)harnstoffe der Formel (I) zeichnen sich durch starke herbizide Wirksamkeit aus.

Ueberraschenderweise zeigen die neuen Verbindungen der Formel (I) erheblich bessere herbizide Wirkung als vorbekannte Harnstoff-Derivate gleicher Wirkungsrichtung.

Gegenstand der Erfindung sind vorzugsweise Verbindungen der Formel (I), in welcher

Q für Sauerstoff steht,

$R^1$ für Wasserstoff oder Methyl steht,

$R^2$ für Wasserstoff, Fluor, Chlor, Brom, Methyl, Trifluormethyl, Methoxy, Ethoxy, Difluormethoxy, Methylthio, Ethylthio, Amino, Methylamino, Ethylamino, Dimethylamino oder Diethylamino steht,

X für Stickstoff oder eine -CH-Gruppierung steht,

Y für Stickstoff oder eine $-CR^3$-Gruppierung steht,

worin

$R^3$ für Wasserstoff, Fluor, Chlor, Brom, Methyl oder Acetyl steht,

und

Z für Stickstoff oder eine $-CR^4$-Gruppierung steht,

worin

$R^4$ für Wasserstoff, Fluor, Chlor, Brom, Methyl, Ethyl, Methoxy, Ethoxy, Propoxy, Isopropoxy, Methylthio, Ethylthio, Methylamino, Ethylamino, Dimethylamino oder Diethylamino steht.

Gegenstand der Erfindung sind insbesondere Verbindungen der Formel (I), in welcher

Q für Sauerstoff steht,

$R^1$ für Wasserstoff steht,

$R^2$ für Methyl, Methoxy, Ethoxy oder Dimethylamino steht,

X für Stickstoff steht,

Y für Stickstoff oder eine $-CR^3$-Gruppierung steht,

worin

$R^3$ für Wasserstoff steht,

und

Z für eine $-CR^4$-Gruppierung steht,

worin

$R^4$ für Wasserstoff, Methyl, Methoxy oder Ethoxy steht.

Verwendet man beispielsweise für die Verfahrensvariante (a) 2-Trifluormethoxy-phenylsulfonyl-isothiocyanat und 2-Amino-4-ethoxy-6-methyl-s-triazin als Ausgangsstoffe, so kann der Reaktionsablauf durch folgendes Formelschema skizziert werden:

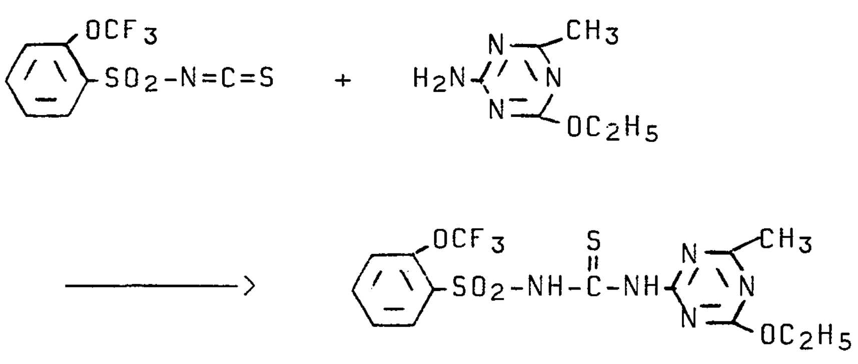

Verwendet man beispielsweise für die Verfahrensvariante (b) 2-Trifluormethoxy-benzolsulfonsäureamid und O-Phenyl-N-(4-ethylamino-6-methylthio-s-triazin-2-yl)-urethan als Ausgangsstoffe, so kann der Reaktionsablauf durch das

folgende Formelschema skizziert werden:

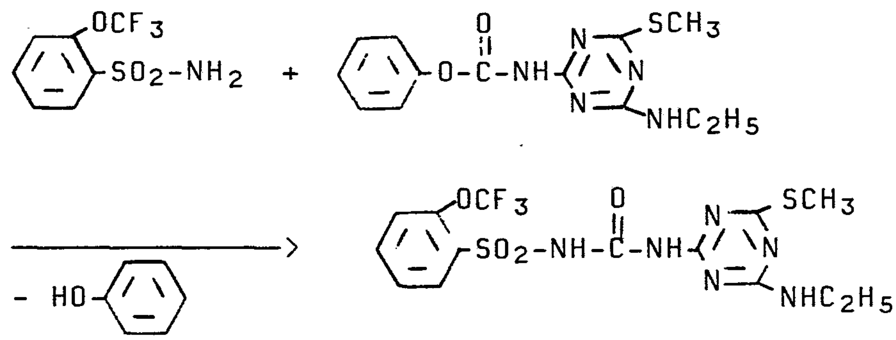

Verwendet man beispielsweise für die Verfahrensvariante (c) O-Phenyl-N-(2-trifluormethoxy-phenylsulfonyl)-urethan und 2-Amino-4-chlor-6-methoxy-pyrimidin als Ausgangstoffe, so kann der Reaktionsablauf durch das folgende Formelschema skizziert werden:

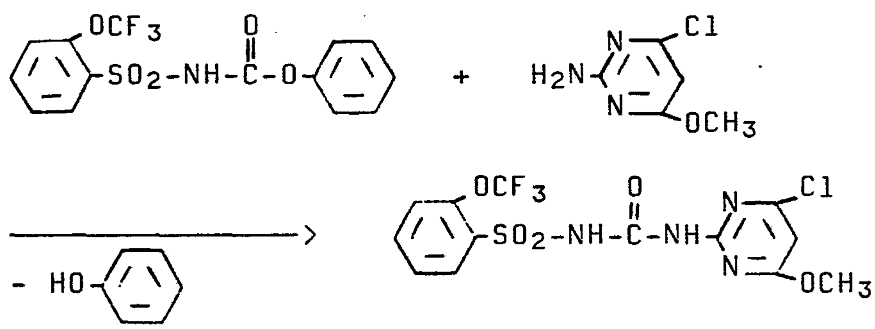

Die als Ausgangsstoffe für die Verfahrensvariante (a) zu verwendenden 2-Trifluormethoxy-phenylsulfonyl-iso(thio)-cyanate sind durch die Formel (II) definiert. In Formel (II) hat Q vorzugsweise bzw. insbesondere die gleiche Bedeutung, wie sie oben im Rahmen der Substituentendefinition für Formel (I) vorzugsweise bzw. als insbesondere bevorzugt angegeben ist.

Als Beispiele für die Verbindungen der Formel (II) seien 2-Trifluormethoxy-phenylsulfonyl-isocyanat und 2-Trifluormethoxy-phenylsulfonyl-isothiocyanat genannt.

Die Verbindungen der Formel (II) sind bisher nicht in der Literatur beschrieben. Man erhält die Verbindung der Formel (II), in welcher Q für Sauerstoff steht, wenn man 2-Trifluormethoxy-benzolsulfonsäureamid der Formel (IV)

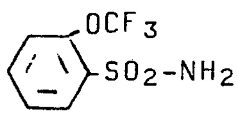

(IV)

mit Thionylchlorid bei Temperaturen zwischen 50 °C und 100 °C umsetzt und anschließend mit Phosgen, gegebenenfalls in Gegenwart eines Verdünnungsmittels, wie z. B. Toluol, und gegebenenfalls in Gegenwart eines Katalysators, wie z. B. Pyridin, bei Temperaturen zwischen 50 °C und 120 °C umsetzt (vergl. J. Org. Chem. 34 (1969), 3200).

Man erhält die Verbindung der Formel (II), in welcher Q für Schwefel steht, wenn man 2-Trifluormethoxy-benzolsulfonsäureamid der Formel (IV) mit Schwefelkohlenstoff in Gegenwart eines Säureakzeptors, wie z. B. Kaliumhydroxid, und gegebenenfalls in Gegenwart eines Verdünnungsmittels, wie z. B. Dimethylformamid, bei Temperaturen zwischen 0 °C und 50 °C umsetzt und anschließend mit Phosgen oder Thionylchlorid, gegebenenfalls in Gegenwart eines Verdünnungsmittels, wie z. B. Methylenchlorid, bei Temperaturen zwischen 0 °C und 50 °C umsetzt (vergl. Arch. Pharm. 299 (1966), 174).

Die weiter als Ausgangsstoffe für die Verfahrensvariante

(a) zu verwendenden Heteroarylamine sind durch die Formel (III) allgemein definiert. In Formel (III) haben $R^1$, $R^2$, X, Y und Z vorzugsweise bzw. insbesondere die gleichen Bedeutungen, wie sie oben im Rahmen der Substituentendefinition für Formel (I) vozugsweise bzw. als insbesondere bevorzugt genannt sind.

Als Beispiele für die Verbindungen der Formel (III) seien genannt:

4,6-Dimethyl-, 4-Methoxy-6-methyl-, 4-Ethoxy-6-methyl- und 4,6-Dimethoxy-2-amino-pyrimidin, 4,6-Dimethyl-, 4-Methoxy-6-methyl-, 4-Ethoxy-6-methyl- und 4,6-Dimethoxy-2-amino-s-triazin sowie 5,6-Dimethyl-2-amino-1,3,4-triazin.

Die Verbindungen der Formel (III) sind bekannt und können nach an sich bekannten Verfahren hergestellt werden (vergl. Chem. Pharm. Bull. 11 (1963), 1382 und US-PS 4 299 960).

Das als Ausgangsstoff für die Verfahrensvariante (b) zu verwendende 2-Trifluormethoxy-benzolsulfonsäureamid der Formel (IV) ist bereits bekannt (vergl. Zh. Org. Khim. [J. Org. Chem. USSR], 8 (1972), 1023 - 1026 [engl. 1032 - 1034]).

Die bei Verfahren (b) weiter als Ausgangsstoffe zu verwendenden N-Heteroaryl-urethane sind durch die Formel (V) allgemein definiert. In Formel (V) haben $R^1$, $R^2$,X, Y und Z vorzugsweise bzw. insbesondere die gleichen Bedeutungen, wie sie oben im Rahmen der Substituentendefinition für Formel (I) vorzugsweise bzw. als insbesondere bevorzugt angegeben sind; ferner stehen Q vorzugsweise für Sauerstoff und $R^5$ für Methyl, Benzyl oder Phenyl, insbesondere für Methyl oder Phenyl.

Als Beispiele für die Verbindungen der Formel (V) seien genannt:

N-(4,6-Dimethyl-pyrimidin-2-yl)-, N-(4-Methoxy-6-methyl-pyrimidin-2-yl)-, N-(4-Ethoxy-6-methyl-pyrimidin-2-yl)-, N-(4,6-Dimethoxy-pyrimidin-2-yl)-, N-(4,6-Dimethyl-s-triazin-2-yl)-, N-(4-Methoxy-6-methyl-s-triazin-2-yl)-, N-(4-Ethoxy-6-methyl-s-triazin-2-yl)-, N-(4,6-Dimethoxy-s-triazin-2-yl)- und N-(5,6-Dimethyl-1,3,4-triazin-2-yl)-O-methyl-urethan bzw. -O-phenyl-urethan.

Die Verbindungen der Formel (V) sind bekannt und können nach an sich bekannten Verfahren hergestellt werden (vergl. EP-OS 101 670).

Die als Ausgangsstoffe für die Verfahrensvariante (c) zu verwendenden N-(2-Trifluormethoxy-phenylsulfonyl)-urethane sind durch die Formel (VI) allgemein definiert. In Formel (VI) stehen vorzugsweise Q für Sauerstoff und $R^6$ für Methyl, Benzyl oder Phenyl, insbesondere für Methyl oder Phenyl.

Als Beispiele für die Verbindungen der Formel (VI) seien genannt:

N-(2-Trifluormethoxy-phenylsulfonyl)-O-methyl-urethan und N-(2-Trifluormethoxy-phenylsulfonyl)-O-phenyl-urethan.

Die Verbindungen der Formel (VI) sind noch nicht in der Literatur beschrieben. Man erhält diese Verbindungen, wenn man 2-Trifluormethoxy-benzolsulfonsäureamid der Formel (IV) mit Verbindungen der Formel (VII)

$$A-\overset{\overset{\displaystyle Q}{\parallel}}{C}-O-R^6 \qquad (VII)$$

in welcher

Q und $R^6$ die oben angegebenen Bedeutungen haben und

A für Chlor oder Phenoxy steht,

gegebenenfalls in Gegenwart von Säureakzeptoren, wie z. B. Natriumhydrid, Kalium-tert.-butylat oder Diazabicycloundecen (DBU), und gegebenenfalls in Gegenwart von Verdünnungsmitteln, wie z. B. Acetonitril oder Dimethylformamid, bei Temperaturen zwischen 0 °C und 50 °C umsetzt (vergl. EP-OS 101 407).

Die Ausgangsprodukte der Formeln (IV) und (VII) sind bekannt.

Das oben unter (a) beschriebene Herstellungsverfahren für die Verbindungen der Formel (I) wird vorzugsweise in Gegenwart von Verdünnungsmitteln durchgeführt. Als solche kommen praktisch alle inerten organischen Solventien in Betracht. Hierzu gehören insbesondere aliphatische oder aromatische, gegebenenfalls halogenierte Kohlenwasserstoffe, wie beispielsweise Pentan, Hexan, Heptan, Benzin, Petrolether, Ligroin, Cyclohexan, Benzol, Toluol, Xylol, Chlorbenzol, Methylenchlorid, Chloroform, Tetrachlormethan, Ether, wie Diethylether, Diisopropylether, Tetrahydrofuran, 1,2-Dimethoxyethan und Dioxan, Ketone, wie Aceton, Methylethylketon und Methylisobutylketon, Nitrile, wie Acetonitril und Propionitril, Ester, wie Essigsäuremethylester und Essigsäureethylester, Amide, wie Dimethylformamid und Dimethylacetamid sowie Dimethylsulfoxid.

Verfahren (a) wird gegebenenfalls in Gegenwart eines Katalysators durchgeführt. Hierzu gehören insbesondere aliphatische, aromatische oder heterocyclische Amine, wie Triethylamin, N,N-Dimethylanilin, Pyridin, 2-Methyl-5-ethylpyridin, 4-Dimethylamino-pyridin, Diazabicyclooctan (DABCO), Diazabicyclononen (DBN) und Diazabicycloundecen (DBU).

Die Reaktionstemperatur kann bei Verfahren (a) innerhalb eines größeren Bereiches variiert werden. Im allgemeinen arbeitet man bei Temperaturen zwischen 0 °C und 150 °C, vorzugsweise zwischen 20 °C und 100 °C.

Zur Durchführung des erfindungsgemäßen Verfahrens (a) setzt man je Mol Heteroarylamin der Formel (III) im allgemeinen 1,0 bis 1,5 Mol, vorzugsweise 1,0 bis 1,2 Mol 2-Trifluormethoxy-phenylsulfonyl-iso(thio)cyanat der Formel (II) ein.

Die Ausgangsstoffe der Formeln (II) und (III) und gegebenenfalls der Katalysator und das Verdünnungsmittel werden im allgemeinen bei Raumtemperatur oder unter leichter Außenkühlung zusammengegeben und das Reaktionsgemisch wird gegebenenfalls bei erhöhter Temperatur bis zum Reaktionsende gerührt.

Die Aufarbeitung und Isolierung der neuen Verbindungen der Formel (I) erfolgt nach üblichen Methoden:
Soweit die Verbindungen der Formel (I) kristallin anfallen, werden sie durch Absaugen isoliert. Andernfalls wird - gegebenenfalls nach Einengen - Wasser und ein mit Wasser praktisch nicht mischbares organisches Lösungsmittel dazugegeben, nach Durchschütteln die organische Phase abgetrennt, getrocknet, filtriert und eingeengt, wobei die Produkte der Formel (I) im Rückstand verbleiben.

Das oben unter (b) beschriebene Herstellungsverfahren für die Verbindungen der Formel (I) wird vorzugsweise in Gegenwart von Verdünnungsmitteln durchgeführt. Es kommen hierbei die gleichen organischen Lösungsmittel in Betracht, die oben im Zusammenhang mit der Beschreibung des erfindungsgemäßen Verfahrens (a) genannt wurden.

Verfahren (b) wird vorzugsweise in Gegenwart eines Säureakzeptors durchgeführt. Als solche kommen alle üblichen anorganischen Säurebindemittel und organischen Basen in Betracht. Hierzu gehören beispielsweise Alkalimetall- und Erdalkalimetall-hydroxide, wie Natrium-, Kalium- und Calcium-hydroxid, Alkalimetall- und Erdalkalimetall-carbonate, wie Natrium-, Kalium- und Calcium-carbonat, sowie aliphatische, aromatische und heterocyclische Amine, wie Triethylamin, N,N'-Dimethylanilin, N,N-Dimethyl-benzylamin, Pyridin, 2-Methyl-5-ethyl-pyridin, 4-Dimethylamino-pyridin, Diazabicyclooctan (DABCO), Diazabicyclononen (DBN) und Diazabicycloundecen (DBU).

Die Reaktionstemperatur kann bei Verfahren (b) innerhalb eines größeren Bereichs variiert werden. Im allgemeinen arbeitet man bei Temperaturen zwischen -20 °C und +100 °C, vorzugsweise zwischen 0 °C und 50 °C.

Zur Durchführung des erfindungsgemäßen Verfahrens (b) setzt man je Mol N-Heteroaryl-urethan der Formel (V) im allgemeinen 1,0 bis 1,5 Mol, vorzugsweise 1,0 bis 1,2 Mol 2-Trifluormethoxy-benzolsulfonsäureamid der Formel (IV) ein.

Die Ausgangsstoffe der Formeln (IV) und (V) und gegebenenfalls der Säureakzeptor und das Verdünnungsmittel werden im allgemeinen bei Raumtemperatur oder unter leichter Aussenkühlung zusammengegeben und das Reaktionsgemisch wird gegebenenfalls bei erhöhter Temperatur bis zum Reaktionsende gerührt. Die Aufarbeitung und Isolierung der neuen Verbindungen der Formel (I) kann nach üblichen Methoden durchgeführt werden:
Im allgemeinen wird - gegebenenfalls nach Einengen - mit Wasser verrührt und gegebenenfalls mit Salzsäure angesäuert. Soweit die Verbindungen der Formel (I) hierbei kristallin anfallen, werden sie durch Absaugen isoliert. Andernfalls wird mit einem mit Wasser praktisch nicht mischbaren Lösungsmittel, wie z. B. Essigester oder Methylenchlorid extrahiert, die organische Phase abgetrennt, getrocknet, filtriert und eingeengt, wobei die Produkte der Formel (I) im Rückstand verbleiben.

Das oben unter (c) beschriebene Herstellungsverfahren für die Verbindungen der Formel (I) wird vorzugsweise in Gegenwart von Verdünnungsmitteln durchgeführt. Es kommen hierbei die gleichen organischen Lösungsmittel in Betracht, die oben im Zusammenhang mit der Beschreibung des erfindungsgemäßen Verfahrens (a) genannt wurden.

Verfahren (c) wird vorzugsweise in Gegenwart eines Säureakzeptors durchgeführt. Als solche kommen hierbei die gleichen anorganischen Säurebindemittel und organischen Basen in Betracht, die oben im Zusammenhang mit der Beschreibung des erfindungsgemäßen Verfahrens (b) genannt wurden.

Die Reaktionstemperatur kann bei Verfahren (c) innerhalb

eines größeren Bereichs variiert werden. Im allgemeinen arbeitet man bei Temperaturen zwischen -20 °C und + 100 °C, vorzugsweise zwischen 0 °C und 50 °C.

Zur Durchführung des erfindungsgemäßen Verfahrens (c) setzt man je Mol N-(2-Trifluormethoxy-phenylsulfonyl)-urethan der Formel (VI) im allgemeinen 1,0 bis 1,5 Mol, vorzugsweise 1,0 bis 1,2 Mol Heteroarylamin der Formel (III) ein.

Die Durchführung und Aufarbeitung kann bei Verfahren (c) wie oben für Verfahren (b) beschrieben, erfolgen.

Die erfindungsgemäßen Wirkstoffe können als Defoliants, Desiccants, Krautabtötungsmittel und insbesondere als Unkrautvernichtungsmittel verwendet werden. Unter Unkraut im weitesten Sinne sind alle Pflanzen zu verstehen, die an Orten aufwachsen, wo sie unerwünscht sind. Ob die erfindungsgemäßen Stoffe als totale oder selektive Herbizide wirken, hängt im wesentlichen von der angewendeten Menge ab.

Die erfindungsgemäßen Wirkstoffe können z.B. bei den folgenden Pflanzen verwendet werden:

Dikotyle Unkräuter der Gattungen: Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Rotala, Lindernia, Lamium, Veronica, Abutilon, Emex, Datura, Viola, Galeopsis, Papaver, Centaurea.

Dikotyle Kulturen der Gattungen: Gossypium, Glycine, Beta, Daucus, Phaseolus, Pisum, Solanum, Linum, Ipomoea, Vicia, Nicotiana, Lycopersicon, Arachis, Brassica, Lactuca, Cucumis, Cucurbita.

Monokotyle Unkräuter der Gattungen: Echinochloa, Setaria, Panicum, Digitaria, Phleum, Poa, Festuca, Eleusine, Brachiaria, Lolium, Bromus, Avena, Cyperus, Sorghum, Agropyron, Cynodon, Monochoria, Fimbristylis, Sagittaria, Eleocharis, Scirpus, Paspalum, Ischaemum, Sphenoclea, Dactyloctenium, Agrostis, Alopecurus, Apera.

Monokotyle Kulturen der Gattungen: Oryza, Zea, Triticum, Hordeum, Avena, Secale, Sorghum, Panicum, Saccharum, Ananas, Asparagus, Allium.

Die Verwendung der erfindungsgemäßen Wirkstoffe ist jedoch keineswegs auf diese Gattungen beschränkt, sondern erstreckt sich in gleicher Weise auch auf andere Pflanzen.

Die Verbindungen eignen sich in Abhängigkeit von der Konzentration zur Totalunkrautbekämpfung z.B. auf Industrie- und Gleisanlagen und auf Wegen und Plätzen mit und ohne Baumbewuchs. Ebenso können die Verbindungen zur Unkrautbekämpfung in Dauerkulturen, z.B. Forst-, Ziergehölz-, Obst-, Wein-, Citrus-, Nuß-, Bananen-, Kaffee-, Tee-, Gummi-, Ölpalm-, Kakao-, Beerenfrucht- und Hopfenanlagen und zur selektiven Unkrautbekämpfung in einjährigen Kulturen eingesetzt werden.

Die erfindungsgemäßen Wirkstoffe können im Vor- und im Nachauflaufverfahren zur Bekämpfung mono- und dikotyler Unkräuter eingesetzt werden.

Die Wirkstoffe können in die üblichen Formulierungen überführt werden, wie Lösungen, Emulsionen, Spritzpulver, Suspensionen, Pulver, Stäubemittel, Pasten, lösliche Pulver, Granulate, Suspensions-Emulsions-Konzentrate, wirkstoffimprägnierte Natur- und synthetische Stoffe sowie Feinstverkapselungen in polymeren Stoffen.

Diese Formulierungen werden in bekannter Weise hergestellt, z.B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln.

Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten und chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole, wie Butanol oder Glycol sowie deren Ether und Ester, Ketone wie Aceton, Methylethylketon, Methylisobutylketon oder Cyclohexanon, stark polare Lösungsmittel, wie Dimethylformamid und Dimethylsulfoxid, sowie Wasser.

Als feste Trägerstoffe kommen in Frage:
z. B. Ammoniumsalze und natürliche Gesteinsmehle, wie Kaoline, Tonerden, Talkum, Kreide, Quarz, Attapulgit,

Montmorillonit oder Diatomeenerde und synthetische Gesteinsmehle, wie hochdisperse Kieselsäure, Aluminiumoxid und Silikate, als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengeln; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylaryl-polyglykolether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulvrige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können als solche oder in ihren Formulierungen auch in Mischung mit bekannten Herbiziden zur Unkrautbekämpfung Verwendung finden, wobei Fertigformulierungen oder Tankmischungen möglich sind.

Für die Mischungen kommen bekannte Herbizide wie z.B. N-(2-Benzthiazolyl)-N,N'-dimethyl-harnstoff, 3-(3-Chlor-4-methylphenyl)-1,1-dimethylharnstoff, 3-(4-isopropylphenyl)-1,1-dimethylharnstoff, 4-Amino-6-(1,1-dimethylethyl)-3-methylthio-1,2,4-triazin-5(4H)-on, 4-Amino-6-(1,1-dimethyl-ethyl)-3-ethylthio-1,2,4-triazin-5(4H)-on, 1-Amino-6-ethylthio-3-(2,2-dimethylpropyl)-1,3,5-triazin-2,4-(1H, 3H)-dion, 4-Amino-3-methyl-6-phenyl-1,2,4-triazin-5(4H)-on, 2-Chlor-4-ethylamino-6-isopropyl-amino-1,3,5-triazin, das R-Enantiomere des 2-/4-(3,5-Dichlor-pyridin-2-oxy)-phenoxy7-propionsäure-(trimethylsilyl)-methylesters, das R-Enantiomere des 2-/4-(3,5-Dichlorpyridyl-2-oxy)-phenoxy7-propionsäure-(2-benzyloxy)-ethylesters, 2,4-Dichlorphenoxyessigsäure, 2-(2,4-Dichlorphenoxy)-propionsäure, 4-Chlor-2-methyl-phenoxy-essigsäure, 2-(2-Methyl-4-chlor-phenoxy)-propionsäure, 3,5-Dijod-4-hydroxy-benzonitril, 3,5-Dibrom-4-hydroxy-benzonitril sowie Diphenylether und Phenylpyridazine, wie z.B. Pyridate, 2-Chlor-N-(2-ethyl-6-methylphenyl)-N-(2-methoxy-1-methylethyl)-acetamid, 2-Chlor-N-(2,6-diethylphenyl)-N-(methoxymethyl)-acetamid, 2-(2-Benzothiazolyloxy)-N-methyl-N-phenylacetamid, N-Phosphonomethylglycin sowie 3-(1-Methylethyl)-1H-2,1,3-benzothiadiazin-4(3H)-on-2,2-dioxid. Einige Mischungen zeigen überraschenderweise auch synergistische Wirkung.

Auch eine Mischung mit anderen bekannten Wirkstoffen, wie Fungiziden, Insektiziden, Akariziden, Nematiziden, Schutzstoffen gegen Vogelfraß, Pflanzennährstoffen und Bodenstrukturverbesserungsmitteln ist möglich.

Die Wirkstoffe können als solche, in Form ihrer Formulierungen oder den daraus durch weiteres Verdünnen bereiteten Anwendungsformen, wie gebrauchsfertige Lösungen, Suspensionen, Emulsionen, Pulver, Pasten und Granulate angewandt werden. Die Anwendung geschieht in üblicher Weise, z.B. durch Gießen, Spritzen, Sprühen, Streuen.

Die erfindungsgemäßen Wirkstoffe können sowohl vor als auch nach dem Auflaufen der Pflanzen appliziert werden.

Sie können auch vor der Saat in den Boden eingearbeitet werden.

Die angewandte Wirkstoffmenge kann in einem größeren Bereich schwanken. Sie hängt im wesentlichen von der Art des gewünschten Effektes ab. Im allgemeinen liegen die Aufwandmengen zwischen 0,001 und 10 kg Wirkstoff pro Hektar Bodenfläche, vorzugsweise zwischen 0,05 und 5 kg pro ha.

Die Herstellung und die Verwendung der erfindungsgemäßen Wirkstoffe geht aus den nachfolgenden Beispielen hervor.

Herstellungsbeispiele

Beispiel 1

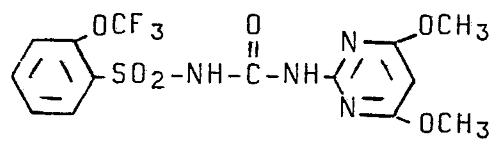

(Verfahren (a))

Eine Lösung von 28 g (0,11 Mol) 2-Trifluormethoxyphenylsulfonylisocyanat in 30 ml Toluol wird zu einer Mischung aus 15,5 g (0,10 Mol) 2-Amino-4,6-dimethoxy-pyrimidin und 100 ml Acetonitril bei 20 °C unter Rühren tropfenweise gegeben und das Reaktionsgemisch wird 2 Stunden bei 60 °C gerührt. Nach Abkühlen wird das kristallin angefallene Produkt durch Absaugen isoliert.

Man erhält 20,7 g (49 % der Theorie) 1-(2-Trifluormethoxyphenylsulfonyl)-3-(4,6-dimethoxy-pyrimidin-2-yl)-harnstoff vom Schmelzpunkt 170 °C.

Beispiel 2

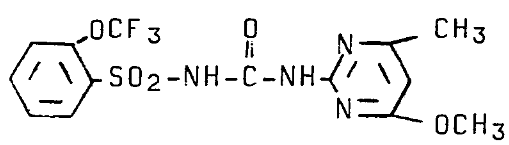

(Verfahren (b))

2,5 g (0,022 Mol) Kalium-tert.-butanolat werden bei 20 °C bis 30 °C portionsweise unter Rühren zu einer Mischung aus

4,0 g (0,019 Mol) 2-Trifluormethoxybenzolsulfonsäureamid, 5,0 g (0,019 Mol) N-(4-Methoxy-6-methyl-pyrimidin-2-yl)-O-phenyl-urethan und 50 ml Acetonitril gegeben. Dann wird eingeengt, der Rückstand in 30 ml Wasser aufgenommen und mit konzentrierter Salzsäure angesäuert. Das hierbei kristallin angefallene Produkt wird durch Absaugen isoliert.

Man erhält 1,2 g (15 % der Theorie) 1-(2-Trifluormethoxyphenylsulfonyl)-3-(4-methoxy-6-methyl-pyrimidin-2-yl)-harnstoff vom Schmelzpunkt 162 °C.

Nach den in den vorausgehenden Beispielen exemplarisch beschriebenen Verfahren können die in der nachstehenden Tabelle 1 aufgeführten Verbindungen der Formel (I) hergestellt werden:

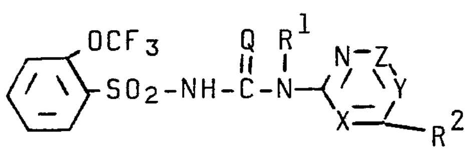

(I)

Tabelle 1

| Beispiel Nr. | Q | $R^1$ | $R^2$ | X | Y | Z | Schmelzpunkt (°C) |
|---|---|---|---|---|---|---|---|
| 3 | O | H | $CH_3$ | N | CH | $C-CH_3$ | 194 |
| 4 | O | H | Cl | N | CH | C-Cl | 164 |
| 5 | O | H | $CH_3$ | N | $C-CH_3$ | N | 134 |
| 6 | O | H | $CH_3$ | N | CH | CH | 180-181 |
| 7 | O | H | $CH_3$ | CH | CH | $C-CH_3$ | 204-205 |
| 8 | O | H | $OCH_3$ | N | N | $C-CH_3$ | 162 |
| 9 | O | H | $CH_3$ | N | N | $C-CH_3$ | 171 |
| 10 | S | H | $CH_3$ | N | CH | $C-CH_3$ | |
| 11 | S | H | $OCH_3$ | N | CH | $C-OCH_3$ | |
| 12 | O | H | $OCH_3$ | N | CH | C-Cl | 158-160 |
| 13 | O | H | $CH_3$ | N | $C-COCH_3$ | CH | |
| 14 | O | H | $CH_3$ | N | N | $C-N(CH_3)_2$ | 178 (Zers.) |
| 15 | O | H | $OCH_3$ | N | N | $C-OCH_3$ | 182-183 |
| 16 | O | H | $CH_3$ | N | $C-COOC_2H_5$ | N | |
| 17 | O | H | $OC_2H_5$ | N | CH | $C-OC_2H_5$ | |

Herstellung von Ausgangsstoffen der Formel (II)

Beispiel (II-1)

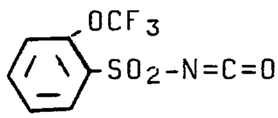

In eine Lösung von 260 g (1,0 Mol) 2-Trifluormethoxybenzolsulfonsäurechlorid in 2 l Tetrahydrofuran wird bei 30 °C etwas mehr als die stöchiometrische Menge Ammoniak eingeleitet. Sobald die Ausfällung von Ammoniumchlorid beendet ist, wird eingeengt, der Rückstand in 2 l Essigsäureethylester aufgeschlämmt und die Mischung mit 1 l Wasser gewaschen. Die organische Phase wird getrocknet, filtriert und eingeengt. Der Rückstand wird mit ca. 500 ml Hexan verrührt und das hierbei kristallin angefallene Produkt durch Absaugen isoliert.

Man erhält 220 g (91 % der Theorie) 2-Trifluormethoxybenzolsulfonsäureamid vom Schmelzpunkt 186 °C.

220 g (0,91 Mol) 2-Trifluormethoxy-benzolsulfonsäureamid werden in 1,5 l Thionylchlorid aufgenommen und nach Zugabe von 5 ml Dimethylformamid wird das Reaktionsgemisch unter Rückfluß zum Sieden erhitzt, bis die Gasentwicklung praktisch beendet ist. Dann wird eingeengt, der Rückstand in 500 ml Toluol, welches 2,5 ml Pyridin enthält, aufgenommen und mit 160 g (1,6 Mol) Phosgen versetzt. Nach 3 Stunden Erhitzen unter Rückfluß wird eingeengt.

Man erhält 225 g (93 % der Theorie) 2-Trifluormethoxyphenylsulfonyl-isocyanat als zähflüssiges Oel, das roh weiter verarbeitet wird.

Beispiel (II-2)

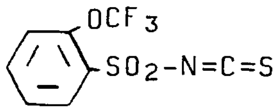

24,1 g (0,1 Mol) 2-Trifluormethoxy-benzolsulfonsäureamid werden in 100 ml Dimethylformamid gelöst. 7,6 g (0,1 Mol) Schwefelkohlenstoff und 5,6 g (0,1 Mol) Kaliumhydroxid-Pulver werden dazugegeben und das Reaktionsgemisch wird bei 35 °C gerührt, bis eine klare Lösung entstanden ist. Dann werden weitere 5,6 g (0,1 Mol) Kaliumhydroxid-Pulver dazu gegeben und das Rühren wird bis zur Bildung einer klaren Lösung fortgesetzt. Dann werden bei 20 °C 100 ml Essigsäureethylester zugetropft. Das hierbei kristallin angefallene Produkt wird durch Absaugen isoliert und unter vermindertem Druck getrocknet. Das getrocknete Produkt wird in 100 ml Toluol suspendiert; bei 0 °C werden 20 g flüssiges Phosgen dazu getropft und das Gemisch wird eine Stunde bei 5 °C bis 10 °C und zwei Stunden bei 20 °C gerührt. Nach Filtration wird eingeengt und man erhält einen öligen Rückstand, welcher im wesentlichen 2-Trifluormethoxy-phenylsulfonyl-isothiocyanat enthält. Das Produkt wird wegen seiner geringen Stabilität möglichst rasch - ohne weitere Reinigung - gemäß dem erfindungsgemäßen Verfahren umgesetzt.

Beispiel A

Pre-emergence-Test

Lösungsmittel: 5 Gewichtsteile Aceton
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Samen der Testpflanzen werden in normalen Boden ausgesät und nach 24 Stunden mit der Wirkstoffzubereitung begossen. Dabei hält man die Wassermenge pro Flächeneinheit zweckmäßigerweise konstant. Die Wirkstoffkonzentration in der Zubereitung spielt keine Rolle, entscheidend ist nur die Aufwandmenge des Wirkstoffs pro Flächeneinheit. Nach drei Wochen wird der Schädigungsgrad der Pflanzen bonitiert in % Schädigung im Vergleich zur Entwicklung der unbehandelten Kontrolle. Es bedeuten:

0 % = keine Wirkung (wie unbehandelte Kontrolle)
100 % = totale Vernichtung

Die erfindungsgemäßen Wirkstoffe zeigen in diesem Test eine sehr gute herbizide Wirksamkeit, insbesondere die Verbindungen aus Herstellungsbeispielen 1, 2, 3, 5, 8.

Beispiel B

Post-emergence-Test

Lösungsmittel: 5 Gewichtsteile Aceton
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Mit der Wirkstoffzubereitung spritzt man Testpflanzen, welche eine Höhe von 5 - 15 cm haben so, daß die jeweils gewünschten Wirkstoffmengen pro Flächeneinheit ausgebracht werden. Die Konzentration der Spritzbrühe wird so gewählt, daß in 2000 l Wasser/ha die jeweils gewünschten Wirkstoffmengen ausgebracht werden. Nach drei Wochen wird der Schädigungsgrad der Pflanzen bonitiert in % Schädigung im Vergleich zur Entwicklung der unbehandelten Kontrolle.
Es bedeuten:

0 % = keine Wirkung (wie unbehandelte Kontrolle)
100 % = totale Vernichtung

Die erfindungsgemäßen Wirkstoffe zeigen in diesem Test eine sehr gute herbizide Wirksamkeit, insbesondere die Verbindungen aus Herstellungsbeispielen 1, 2, 3, 5, 8.

Patentansprüche

1) 1-(2-Trifluormethoxy-phenylsulfonyl)-3-heteroaryl-(thio)harnstoffe der allgemeinen Formel (I)

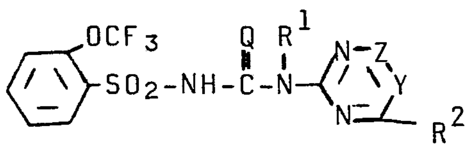

(I)

in welcher

Q für Sauerstoff oder Schwefel steht,

$R^1$ für Wasserstoff, $C_1$-$C_4$-Alkyl oder Benzyl steht,

$R^2$ für Wasserstoff, Halogen, Hydroxy, $C_1$-$C_4$-Alkyl, $C_1$-$C_4$-Halogenalkyl, $C_1$-$C_4$-Alkoxy, $C_1$-$C_4$-Halogenalkoxy, $C_1$-$C_4$-Alkylthio, $C_1$-$C_4$-Halogenalkylthio, Amino, $C_1$-$C_4$-Alkylamino oder Di-($C_1$-$C_4$-alkyl)-amino steht,

X für Stickstoff oder eine -CH-Gruppierung steht,

Y für Stickstoff oder eine -$CR^3$-Gruppierung steht,

worin

$R^3$ für Wasserstoff, Halogen, $C_1$-$C_4$-Alkyl, ($C_1$-$C_2$-Alkyl)-carbonyl oder ($C_1$-$C_2$-Alkoxy)-carbonyl steht,

Z für Stickstoff oder eine $-CR^4$-Gruppierung steht,

worin

$R^4$ für Wasserstoff, Halogen, Hydroxy, $C_1$-$C_4$-Alkyl, $C_1$-$C_4$-Alkoxy, $C_1$-$C_4$-Alkylthio, $C_1$-$C_4$-Alkylamino oder Di-($C_1$-$C_4$-alkyl)-amino steht.

2) Verfahren zur Herstellung von 1-(2-Trifluormethoxy-phenylsulfonyl)-3- heteroaryl -(thio)harnstoffen der allgemeinen Formel (I) gemäß Anspruch 1, dadurch gekennzeichnet, daß man

(a) Trifluormethoxy-phenylsulfonyl-iso(thio)cyanate der Formel (II)

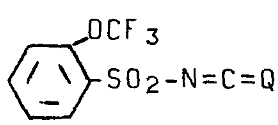

(II)

in welcher

Q für Sauerstoff oder Schwefel steht,

mit Heteroarylaminen der Formel (III)

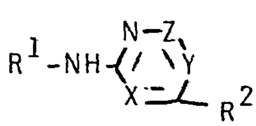

(III)

in welcher

$R^1$, $R^2$ X, Y und Z die oben angegebenen Bedeutungen haben,

gegebenenfalls in Gegenwart von Verdünnungsmitteln und gegebenenfalls in Gegenwart von Katalysatoren umsetzt,

**oder daß man**

(b) 2-Trifluormethoxy-benzolsulfonsäureamid der Formel (IV)

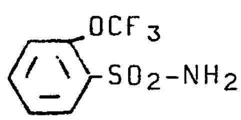

(IV)

mit N-Heteroaryl-urethanen der Formel (V)

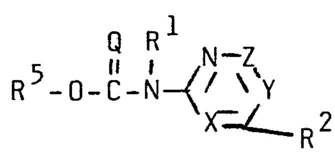

(V)

in welcher

$R^1$, $R^2$, Q, X, Y und Z die oben angegebenen Bedeutungen haben und

$R^5$ für $C_1$-$C_4$-Alkyl, Benzyl oder Phenyl steht,

gegebenenfalls in Gegenwart von Verdünnungsmitteln und gegebenenfalls in Gegenwart von Säureakzeptoren umsetzt,

**oder daß man**

(c) N-(2-Trifluormethoxy-phenylsulfonyl)-urethane der Formel (VI)

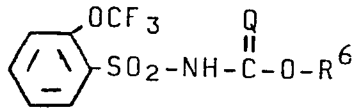

(VI)

in welcher

Q für Sauerstoff oder Schwefel steht und

$R^6$ für $C_1$-C4-Alkyl, Benzyl oder Phenyl steht,

mit Heteroarylaminen der Formel (III)

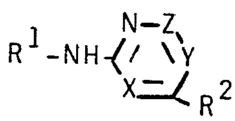

(III)

in welcher

$R^1$, $R^2$, X, Y und Z die oben angegebenen Bedeutungen haben,

gegebenenfalls in Gegenwart von Verdünnungsmitteln und gegebenenfalls in Gegenwart von Säureakzeptoren umsetzt.

3) Herbizide Mittel, gekennzeichnet durch einen Gehalt an mindestens einem 1-(2-Trifluormethoxy-phenylsulfonyl)-3-heteroaryl-(thio)harnstoff der allgemeinen Formel (I) gemäß Anspruch 1.

4) Verwendung von 1-(2-Trifluormethoxy-phenylsulfonyl)-3-heteroaryl-(thio)harnstoffen der allgemeinen Formel (I) gemäß Anspruch 1 zu Bekämpfung von unerwünschtem Pflanzenwachstum.

5) Verfahren zur Herstellung von herbiziden Mitteln, dadurch gekennzeichnet, daß man 1-(2-Trifluormethoxy-phenylsulfonyl)-3-heteroaryl-(thio)harnstoffe der allgemeinen Formel (I) gemäß Anspruch 1 mit Streckmitteln und/oder oberflächenaktiven Mitteln vermischt.

6) 2-Trifluormethoxy-phenylsulfonyl-iso(thio)cyanat der Formel (II)

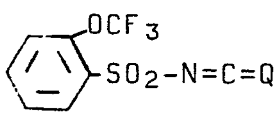

(II)

in welcher

Q für Sauerstoff oder Schwefel steht.

7) Verfahren zur Herstellung von 2-Trifluormethoxy-phenylsulfonyl-iso(thio)cyanat der Formel (II) gemäß Anspruch 6, dadurch gekennzeichnet, daß man 2-Trifluormethoxy-benzolsulfonsäureamid der Formel (IV)

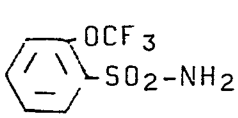

(IV)

a) für den Fall, daß Q für Sauerstoff steht, mit Thionylchlorid bei Temperaturen zwischen 50 °C und 100 °C umsetzt und anschließend mit Phosgen, gegebenenfalls in Gegenwart eines Verdünnungsmittels und gegebenenfalls in Gegenwart eines Katalysators, bei Temperaturen zwischen 50 °C und 120 °C umsetzt;

oder

b) für den Fall, daß Q für Schwefel steht, mit Schwefelkohlenstoff in Gegenwart eines Verdünnungsmittels bei Temperaturen zwischen 0 °C und 50 °C umsetzt und anschließend mit Phosgen oder Thionylchlorid, gegebenenfalls in Gegenwart eines Verdünnungsmittels bei Temperaturen zwischen 0 °C und 50 °C umsetzt.

Europäisches Patentamt

**0 173 312**

# EUROPÄISCHER RECHERCHENBERICHT

EP 85110823.3

| EINSCHLÄGIGE DOKUMENTE | | | KLASSIFIKATION DER ANMELDUNG (Int. Cl.4) |
|---|---|---|---|
| Kategorie | Kennzeichnung des Dokuments mit Angabe, soweit erforderlich, der maßgeblichen Teile | Betrifft Anspruch | |
| A | EP - A3 - 0 044 808 (CIBA)<br>* Zusammenfassung *<br>-- | 1,3,4 | C 07 D 239/42<br>C 07 D 251/42<br>C 07 D 251/48<br>C 07 D 253/04<br>C 07 D 253/06<br>C 07 D 257/08<br>C 07 D 213/75<br>C 07 C 143/828<br>C 07 C 161/04<br>A 01 N 47/36 |
| A | EP - A1 - 0 044 809 (CIBA)<br>* Formel I; Seite 2, Zeile 22 - Seite 5, Zeile 6; Seite 7, Zeilen 22-25; Seite 8, Zeilen 11-16 *<br>-- | 1-7 | |
| A | EP - A3 - 0 094 790 (DU PONT)<br>* Zusammenfassung *<br>-- | 1,3,4 | |
| P,A | EP - A2 - 0 120 814 (CIBA)<br>* Formel I; Ansprüche 18,20,21, 23,27-29; Seite 11, Zeilen 10-14 *<br>-- | 1-7 | **RECHERCHIERTE SACHGEBIETE (Int. Cl.4)** |
| A | DE - A1 - 3 132 944 (CELAMERCK)<br>* Anspruch 1 *<br>-- | 6,7 | C 07 D 239/00<br>C 07 D 251/00<br>C 07 D 253/00<br>C 07 D 257/00<br>C 07 D 213/00<br>C 07 C 143/00<br>C 07 C 161/00 |
| X | EP - A2 - 0 064 322 (DU PONT)<br>* Ansprüche 1,25 *<br>-- | 6,7 | |
| A | US - A - 4 372 778 (LEVITT)<br>* Ansprüche 1,5; Spalte 4, Zeilen 41-60; Spalte 5, Zeilen 35-50 *<br>---- | 1-7 | |

Der vorliegende Recherchenbericht wurde für alle Patentansprüche erstellt.

| Recherchenort | Abschlußdatum der Recherche | Prüfer |
|---|---|---|
| WIEN | 23-10-1985 | HAMMER |

**KATEGORIE DER GENANNTEN DOKUMENTEN**

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503 03 82